# EUROPÄISCHE PATENTANMELDUNG

(11) **EP 3 822 415 A1**
(43) Veröffentlichungstag der Anmeldung: **19.05.2021**
(21) Anmeldenummer: 19209140.3
(22) Anmeldetag: 14.11.2019
(51) Int. Cl.: E02D 3/12, E02D 5/46, G01N 11/06, G01N 33/38

(54) **MESSAUFBAU FÜR EINE RÜCKLAUFZEMENTSUSPENSION, BAUSTELLENANORDNUNG MIT EINEM MESSAUFBAU SOWIE VERFAHREN UND VERWENDUNG**

(71) Anmelder: Keller Holding GmbH, 63067 Offenbach/Main (DE); Stuva e.V., 50827 Köln (DE)
(72) Erfinder: Hinzmann, Uwe, 21386 Betzendorf (DE); Thienert, Christian, 40699 Erkrath (DE); Klaproth, Christoph, 52070 Aachen (DE); Ludwig, Frank, 45134 Essen (DE); Otterbein, Reiner, 58093 Hagen (DE)
(74) Vertreter: Negendanck, Matthias

(57) **Zusammenfassung**

Das Düsenstrahlverfahren (DSV) ist eine Form der Baugrundinjektion zum Erstellen von Zement-Bodengemisch-Körpern im Erdreich. Bei den Düsenstrahlarbeiten im Düsenstrahlverfahren ergibt sich verfahrensbedingt ein Rücklauf, welcher einen hohen Zementanteil aufweist. Dieser Rücklauf wird üblicherweise praktisch ungenutzt entsorgt. Es ist Aufgabe der Erfindung, einen Messaufbau für eine Rücklaufzementsuspension, eine Baustellenanordnung mit einem oder dem Messaufbau und ein Verfahren vorzuschlagen, welcher/welche/welches kostengünstig, zuverlässig und einfach ausführbar ist.

Insbesondere wird ein Messaufbau 13 für eine Rücklaufzementsuspension vorgeschlagen, mit einer Messmischeinrichtung 20 zur Vermischung einer Rücklaufzementsuspension mit einem Zusatzmittel zu einer Testsuspension, mit einem Messstreckenabschnitt 22, mit einer Messvorrichtung 26 zur Messung von mindestens einer Hauptmessgröße, wobei die Hauptmessgröße abhängig von den rheologischen Eigenschaften der Testsuspension ist, und mit einer Auswerteeinrichtung 29, wobei die Auswerteeinrichtung 29 ausgebildet ist, in Abhängigkeit der Hauptmessgröße mindestens eine Qualitätskennzahl für die Rücklaufzementsuspension zu schätzen und/oder zu bestimmen.

## Beschreibung

Die Erfindung betrifft einen Messaufbau für eine Rücklaufzementsuspension. Die Erfindung betrifft auch eine Baustellenanordnung mit einem oder dem Messaufbau sowie ein entsprechendes Verfahren.

Das Düsenstrahlverfahren (DSV) ist eine Form der Baugrundinjektion zum Erstellen von Zement-Bodengemisch-Körpern im Erdreich. In den letzten Jahren wurden in Deutschland Düsenstrahlarbeiten mit einem großen jährlichen Gesamtvolumen ausgeführt. Bei den Düsenstrahlarbeiten im Düsenstrahlverfahren ergibt sich verfahrensbedingt ein Rücklauf, welcher einen hohen Zementanteil aufweist. Dieser Rücklauf wird üblicherweise praktisch ungenutzt entsorgt.

Die Druckschrift DE 690 09 038 T2 (EP 0 440 825 B1), die wohl den nächstkommenden Stand der Technik bildet, beschreibt dagegen ein Verfahren zur Wiederverwendung des Schlamms bei der Bodenstabilisierung, wobei der bei der Bearbeitung abgegebene Rohschlamm gesammelt, aufbereitet und als ein Spritzmaterial, nämlich als Härtemittel, wiederverwendet wird. Es wird hierbei eine magnetische Permeabilität aufweisende Substanz zu dem einzuspritzenden Härtemittel beigefügt, nachfolgend wird das Bodenstabilisierungsverfahren durchgeführt, bei welchem das Härtemittel mit der Substanz in den Boden eingespritzt wird. Der während der Ausführung des Bodenstabilisierungsverfahrens ausgestoßene Rohschlamm wird gesammelt, wobei an dem Rohschlamm ein physikalischer Parameter gemessen wird, welcher die magnetische Permeabilität des Härtemittels in dem Rohschlamm anzeigt, um hieraus die Menge an Härtemittel zu berechnen, welche in dem gesammelten Rohschlamm enthalten ist.

Es ist Aufgabe der Erfindung, einen Messaufbau für eine Rücklaufzementsuspension, sowie eine Baustellenanordnung mit einem Messaufbau für die Rücklaufzementsuspension und ein Verfahren vorzuschlagen, welcher/welche/welches kostengünstig, zuverlässig und einfach ausführbar ist.

Diese Aufgabe wird durch einen Messaufbau mit den Merkmalen des Anspruchs 1, durch eine Baustellenanordnung mit den Merkmalen des Anspruchs 11 sowie durch ein Verfahren mit den Merkmalen des Anspruchs 16 und durch eine Verwendung mit den Merkmalen des Anspruchs 17 gelöst. Bevorzugte oder vorteilhafte Ausführungsformen der Erfindung ergeben sich aus den Unteransprüchen, der nachfolgenden Beschreibung sowie den beigefügten Figuren.

Gegenstand der Erfindung ist ein Messaufbau für eine Rücklaufzementsuspension, insbesondere zur Messung von mindestens einer Hauptmessgröße der Rücklaufzementsuspension .

Die Rücklaufzementsuspension weist Zementanteile und Bodenanteile auf. Insbesondere wird die Rücklaufzementsuspension durch ein Bodenstabilisierungsverfahren im Düsenstrahlverfahren (DSV) gebildet. Bei dem Düsenstrahlverfahren wird mit einem Bohrgerät, welches zum Beispiel als eine Injektionslanze ausgebildet ist, eine Zementsuspension auf Wasserbasis mit einem Zementanteil, in der neben dem Zementanteil auch andere Bestandteile vorhanden sein können, unter hohem Druck ins Erdreich injiziert, wobei ein Druck von 100 bar oder mehreren 100 bar, zum Beispiel 600 bar, überschritten wird. Durch den Düsenstrahl wird das insbesondere anstehende Bodenmaterial aufgeschnitten und mit der Zementsuspension vermischt, die dann mit dem natürlichen Boden als Zuschlagstoff einen betonartigen Körper bildet. Bei dem Düsenstrahlverfahren ergibt sich jedoch ein Rücklauf, welcher zum Beispiel an einem Bohrlochmund austritt. Dieser Rücklauf wird als Rücklaufzementsuspension bezeichnet und weist Zementanteile von der ursprünglichen Zementsuspension und Bodenanteile von dem aufgeschnittenen Boden auf. Alternativ hierzu kann die Rücklaufzementsuspension durch ein Bohrverfahren erzeugt werden, wobei bei einem Bohrvorgang mit dem Bohrgerät die Zementsuspension auf Wasserbasis mit dem Zementanteil injiziert. Dabei kann es sich beispielsweise um ein Niederdruckbohrverfahren handeln. Auch bei dem allgemeinen Bohrverfahren ergibt sich der Rücklauf, welcher zum Beispiel an dem Bohrlochmund austritt. Dieser Rücklauf wird als Rücklaufzementsuspension bezeichnet und weist Zementanteile von der ursprünglichen Zementsuspension und Bodenanteile auf.

Die Rücklaufzementsuspension kann neben dem Zementanteil auch andere Bindemittelanteile aufweisen.

Insbesondere kann die Rücklaufsuspension als eine ggf. verdünnte mineralische Baustoffsuspension ausgebildet sein, die Zementleimanteile von Betonen aufweist, wobei die Zementleimanteile den Zementanteil bilden oder mitbilden.

Der Messaufbau weist optional eine erste Zuführungseinrichtung zur Zuführung der Rücklaufzementsuspension auf. In einer einfachen Ausgestaltung der Erfindung kann die erste Zuführungseinrichtung als eine Speichervorrichtung, wie zum Beispiel ein Reservoir, im Speziellen eine Wanne, ausgebildet sein, wobei in der Speichervorrichtung die Rücklaufzementsuspension angeordnet ist. Alternativ hierzu kann die erste Zuführungseinrichtung auch als eine Zuleitung oder - wie nachfolgend noch ausgeführt wird - als eine Aufnahmevorrichtung ausgebildet sein. Optional bildet die erste Zuführungseinrichtung mit der Rücklaufzementsuspension einen Bestandteil des Messaufbaus.

Der Messaufbau weist optional eine zweite Zuführungseinrichtung zur Zuführung eines Zusatzmittels auf, wobei das Zusatzmittel bevorzugt ausgebildet ist, die rheologischen Eigenschaften der Rücklaufzementsuspension durch Reaktion mit dem Zementanteil der Rücklaufzementsuspension zu ändern. Unter den rheologischen Eigenschaften wird insbesondere die Fließfähigkeit und/oder Viskosität und/oder Fließgrenze der Rücklaufzementsuspension verstanden.

Es ist bevorzugt, dass das Zusatzmittel ausschließlich oder zumindest maßgeblich mit dem Zementanteil, nicht aber mit dem Bodenanteil, insbesondere Feinkornanteilen des Bodens, und/oder nicht mit den weiteren Bindemittelanteilen interagiert oder reagiert.

Es ist ferner bevorzugt, dass das Zusatzmittel zeitnah, d. h. in einem Zeitintervall von weniger als 5 Minuten, insbesondere weniger als 2,5 Minuten, im Speziellen weniger als 1 Minute, die rheologischen Eigenschaften ändert.

Die Änderung der rheologischen Eigenschaften soll bevorzugt nicht-reversibel sein, um eine Verfälschung der Messergebnisse zu vermeiden.

Der Messaufbau weist eine Messmischeinrichtung zur Vermischung der Rücklaufzementsuspension mit dem Zusatzmittel zu einer Testsuspension auf. Beispielsweise kann die erste Zuführungseinrichtung eine erste Dosierpumpeneinrichtung zur Beschleunigung der Rücklaufzementsuspension und die zweite Zuführungseinrichtung eine zweite Dosierpumpeneinrichtung zur Beschleunigung des Zusatzmittels aufweisen, wobei die beschleunigten Bestandteile in einem Mischer, insbesondere in einem Statikmischer, gemischt werden. Der Mischer und/oder die Messmischeinrichtung kann in der einfachsten Ausgestaltung als eine Zusammenführungseinrichtung für die Ströme der Rücklaufzementsuspension und des Zusatzmittels ausgebildet sein. Alternativ oder ergänzend kann die Zusammenführungseinrichtung eine Düse oder Hochdruckdüse zur Einmischung umfassen.

Vorzugsweise werden als Dosierpumpeneinrichtungen Exzenterschneckenpumpen eingesetzt, um eine pulsationsarme Förderung zu gestatten.

Der Messaufbau weist einen Messstreckenabschnitt zur Führung der Testsuspension auf. Der Messstreckenabschnitt ist insbesondere als eine Leitung, vorzugsweise als eine Leitung mit einem konstanten Strömungsquerschnitt ausgebildet. Im Betrieb wird der Messstreckenabschnitt mit der Testsuspension durchflossen.

Der Messaufbau weist eine Messvorrichtung zur Messung von mindestens einer Hauptmessgröße der Testsuspension an, insbesondere in dem Messstreckenabschnitt auf. Die Hauptmessgröße ist bevorzugt abhängig von den rheologischen Eigenschaften der Testsuspension.

Zudem ist eine Auswerteeinrichtung vorgesehen, wobei die Auswerteeinrichtung beispielsweise als ein Softwaremodul oder als eine digitale Datenverarbeitungseinrichtung, insbesondere ein Computer, ein Mikrocontroller oder dergleichen, ausgebildet sein kann. Die Auswerteeinrichtung ist insbesondere programmtechnisch und/oder schaltungstechnisch ausgebildet in Abhängigkeit der Hauptmessgröße mindestens eine Qualitätskennzahl für die Rücklaufzementsuspension zu schätzen und/oder zu bestimmen. Für diese Funktion kann die Auswerteeinrichtung beispielsweise auf ein Regelwerk, Tabellen, insbesondere zur Kalibrierung des Messaufbaus, analytische Funktionen, künstliche neuronale Netze etc. zurückgreifen.

Die Qualitätskennzahl ist bevorzugt ein Maß für den Zementanteil der Rücklaufzementsuspension und/oder die Reaktivität der Rücklaufzementsuspension, insbesondere den reaktiven Zementanteil in der Testsuspension und damit in der Rücklaufzementsuspension. Es ist bekannt, dass sich Zement mit gestörter oder verzögerter Festigkeitsentwicklung auch bereits im noch frischen, d. h. fließfähigen Zustand, weniger reaktiv verhalten, was sich anhand verringerter Hydrationswärmeentwicklung oder vermindertem Ansteifen bemerkbar machen kann. Somit ist die Änderung der rheologischen Eigenschaften der Rücklaufzementsuspension und die daraus abgeleitete Qualitätskennzahl ein Maß für die Reaktivität des Zementanteils in der Rücklaufzementsuspension. Ferner ist die Qualitätskennzahl ein Maß für den Zementanteil in der Rücklaufzementsuspension. Zusammengefasst ist die Qualitätskennzahl insbesondere ein Maß für den reaktiven Zementanteil in der Rücklaufzementsuspension.

Alternativ oder ergänzend betrachtet ist die Qualitätskennzahl bevorzugt ein Maß für die Mischeignung der Rücklaufzementsuspension mit einer insbesondere frischen Zementsuspension zur Erzeugung einer einsatzfähigen Mischzementsuspension beschreibt. Dabei wird davon ausgegangen, dass die Rücklaufzementsuspension durch das Einbringen des Bodenanteils einen verringerten Zementanteil, insbesondere einen verringerten reaktiven Zementanteil aufweist. Durch eine Mischung mit einer Zementsuspension, insbesondere einer frischen Zementsuspension, kann eine Mischzementsuspension hergestellt werden, wobei das Mischverhältnis zwischen der Rücklaufzementsuspension und der Zementsuspension so eingestellt wird, dass die Mischzementsuspension einsatzfähig ist, insbesondere einsatzfähig für das oben beschriebene Düsenstrahlverfahren und/oder Bohrverfahren ist. Diese funktionale Definition der Qualitätskennzahl nimmt die mögliche Verwendung der Qualitätskennzahl auf, wie diese in Zusammenhang mit der Baustellenanordnung beschrieben wird.

Die Erfindung weist gegenüber dem Stand der Technik einige Vorteile auf:
So muss nicht die Rücklaufzementsuspension oder die Zementsuspension in der Gesamtheit mit dem Zusatzmittel vermischt werden, wie dies im Stand der Technik beschrieben ist, sondern vorzugsweise nur ein Messanteil der Rücklaufzementsuspension muss mit dem Zusatzmittel vermischt werden. Dadurch wird signifikant Zusatzmittel eingespart. Ferner belastet das Zusatzmittel aufgrund des geringen Einsatzes die Umwelt nicht.

Der Messaufbau ermöglicht eine Onlinemessung von der Qualitätskennzahl, so dass die Rücklaufzementsuspension in Echtzeit und somit auf der Baustelle in das Düsenstrahlverfahren wieder eingespeist werden kann, so dass sich der Entsorgungsaufwand für die Rücklaufzementsuspension deutlich reduzieren kann.

Es ist dabei eine weiterführende Überlegung der Erfindung, dass durch die Messung der induzierten Änderung der rheologischen Eigenschaften, wobei die Änderungen in Abhängigkeit des Zementanteils, insbesondere des reaktiven Zementanteils, erfolgen, der Zementanteil, insbesondere der reaktive Zementanteil, in der Rücklaufzementsuspension zuverlässig abgeschätzt werden kann und damit das Potenzial vorhanden ist, auf der Baustelle die Rücklaufzementsuspension in das Düsenstrahlverfahren und/oder Bohrverfahren wieder einzuspeisen, wobei durch das Mischungsverhältnis in Abhängigkeit des Zementanteils, insbesondere des reaktiven Zementanteils, sichergestellt ist, dass die Mischzementsuspension einsatzbereit für das Düsenstrahlverfahren bzw. Bohrverfahren ist. Dabei wäre es prinzipiell möglich, den Zementanteil, insbesondere den reaktiven Zementanteil, über physikalische Größen als die Qualitätskennzahl anzugeben. Es ist jedoch auch möglich, die Qualitätskennzahl so zu wählen, dass der Zementanteil, insbesondere der reaktive Zementanteil, qualitativ beschrieben wird.

Es ist besonders bevorzugt vorgesehen, dass der Messaufbau kontinuierlich betreibbar ist. Somit kann die mindestens eine Qualitätskennzahl in Echtzeit zu der durch den Messstreckenabschnitt fließenden Testsuspension und damit in Echtzeit für die Rücklaufzementsuspension ausgegeben werden.

Bei einer bevorzugten Ausgestaltung der Erfindung ist die Hauptmessgröße als eine strömungstechnische Kenngröße, insbesondere Änderung einer strömungstechnischen Kenngröße, im Speziellen als eine Druckänderung und/oder als eine Änderung der Strömungsgeschwindigkeit in dem Messstreckenabschnitt ausgebildet. Die strömungstechnische Kenngröße, insbesondere die Druckänderung bzw. Fließgeschwindigkeit, entsteht oder verändert sich in dem Messstreckenabschnitt in Abhängigkeit der rheologischen Eigenschaften der Testsuspension und damit der Rücklaufzementsuspension, insbesondere in Abhängigkeit der Fließfähigkeit und/oder der Viskosität und/oder der Fließgrenze der Testsuspension bzw. der Rücklaufzementsuspension. Besonders bevorzugt ist die Druckänderung als eine Druckerhöhung bzw. Erhöhung der Strömungsgeschwindigkeit ausgebildet, bei alternativen Ausgestaltungen kann das Zusatzmittel auch so gewählt werden, dass eine Druckreduktion bzw. Erniedrigung der Fließgeschwindigkeit auftritt.

Es ist möglich, dass vor, innerhalb und/oder nach dem Messstreckenabschnitt aktive und/oder passive strömungstechnische Elemente angeordnet sind, die dazu führen, dass sich die strömungstechnische Kenngröße der in Abhängigkeit der rheologischen Eigenschaften der Testsuspension und damit der Rücklaufzementsuspension, insbesondere in Abhängigkeit der Fließfähigkeit und/oder der Viskosität und/oder der Fließgrenze der Testsuspension bzw. der Rücklaufzementsuspension ändert.

Die passiven strömungstechnischen Elemente können beispielsweise als Drossel, Umlenkung etc. ausgebildet sein, die aktiven strömungstechnischen Elemente können als Pumpe, insbesondere als Strömungsmaschine realisiert sein.

Bei einer bevorzugten konstruktiven Ausgestaltung der Erfindung weist der Messaufbau einen Zuführungsabschnitt zur Zuführung der Testsuspension zu dem Messstreckenabschnitt und einen Abführungsabschnitt zur Abführung der Testsuspension aus dem Messstreckenabschnitt auf. Insbesondere bilden der Zuführungsabschnitt, der Messstreckenabschnitt und Abführungsabschnitt eine geschlossene Leitung.

Insbesondere ist der Messstreckenabschnitt mit verengtem Strömungsquerschnitt im Vergleich zu dem Zuführungsabschnitt und zu dem Abführungsabschnitt ausgebildet. Es wird insbesondere beansprucht, dass der Strömungsquerschnitt des Messstreckenabschnitts an mindestens einer Stelle kleiner als der Strömungsquerschnitt des Zuführungsabschnitts ausgebildet ist. Ferner ist der Strömungsquerschnitt des Messstreckenabschnitts an mindestens einer Stelle kleiner als der Strömungsquerschnitt des Abführungsabschnitts ausgebildet. Vorzugsweise weist der Messstreckenabschnitt einen konstanten Strömungsabschnitt auf, wobei der konstante Strömungsabschnitt kleiner als der Strömungsabschnitt des Zuführungsabschnitts und des Abführungsabschnitts ausgebildet ist.

Alternativ oder ergänzend ist die Strömungsgeschwindigkeit in dem Messstreckenabschnitt größer als oder gleich groß wie die Strömungsgeschwindigkeit in dem Zuführungsabschnitt sowie die Strömungsgeschwindigkeit in dem Abführungsabschnitt.

Alternativ oder ergänzend ist strömungstechnisch zwischen Zuführungsabschnitt und dem Messstreckenabschnitt eine Eingangsdrossel zur Verringerung des Strömungsquerschnitts und zwischen Messstreckenabschnitt und dem Abführungsabschnitt eine Ausgangsdrossel zur Vergrößerung des Strömungsquerschnitts angeordnet.

Der Strömungsquerschnitt von dem Zuführungsabschnitt und von dem Abführungsabschnitt kann gleich ausgebildet sein, er kann jedoch auch unterschiedlich realisiert sein.

Alle genannten Alternativen setzen insbesondere die Idee um, über die Änderung der strömungstechnischen Kenngröße, insbesondere die Druckänderung, insbesondere die Druckerhöhung, und/oder Änderung der Strömungsgeschwindigkeit in dem Messstreckenabschnitt die rheologischen Eigenschaften der Testsuspension, insbesondere die Fließfähigkeit und/oder Viskosität und/oder die Fließgrenze ermitteln oder abschätzen zu können.

Alternativ ist der Messstreckenabschnitt mit vergrößertem oder gleichem Strömungsquerschnitt im Vergleich zu dem Zuführungsabschnitt und zu dem Abführungsabschnitt ausgebildet. Es wird insbesondere beansprucht, dass der Strömungsquerschnitt des Messstreckenabschnitts an mindestens einer Stelle größer als der Strömungsquerschnitt des Zuführungsabschnitts ausgebildet ist. Ferner ist der Strömungsquerschnitt des Messstreckenabschnitts an mindestens einer Stelle größer als der Strömungsquerschnitt des Abführungsabschnitts ausgebildet. Vorzugsweise weist der Messstreckenabschnitt einen konstanten Strömungsabschnitt auf, wobei der konstante Strömungsabschnitt größer als der Strömungsabschnitt des Zuführungsabschnitts und des Abführungsabschnitts ausgebildet ist.

Alternativ oder ergänzend ist die Strömungsgeschwindigkeit in dem Messstreckenabschnitt kleiner als oder gleich groß wie die Strömungsgeschwindigkeit in dem Zuführungsabschnitt sowie die Strömungsgeschwindigkeit in dem Abführungsabschnitt.

Alternativ oder ergänzend ist strömungstechnisch zwischen Zuführungsabschnitt und dem Messstreckenabschnitt eine Eingangsdrossel zur Vergrößerung des Strömungsquerschnitts und zwischen Messstreckenabschnitt und dem Abführungsabschnitt eine Ausgangsdrossel zur Verkleinerung des Strömungsquerschnitts angeordnet.

Der Strömungsquerschnitt von dem Zuführungsabschnitt und von dem Abführungsabschnitt kann gleich ausgebildet sein, er kann jedoch auch unterschiedlich realisiert sein.

Alle genannten Alternativen setzen insbesondere die Idee um, über die Änderung der strömungstechnischen Kenngröße, insbesondere Druckänderung, insbesondere die Druckerniedrigung, und/oder Änderung der Strömungsgeschwindigkeit in dem Messstreckenabschnitt die rheologischen Eigenschaften der Testsuspension, insbesondere die Fließfähigkeit und/oder Viskosität und/oder die Fließgrenze ermitteln oder abschätzen zu können.

Bei weiteren Alternativen der Erfindung kann nur eine Drossel, wahlweise vor oder nach dem Messstreckenabschnitt eingesetzt werden, wobei die eine Drossel den Strömungsquerschnitt ändert. Es ist auch möglich, dass als strömungstechnisches Element eine Pumpe, insbesondere eine Strömungsmaschine, eingesetzt wird, wobei in dem strömungstechnisch nachfolgenden Messstreckenabschnitt die strömungstechnische Messgröße und deren Änderung erfasst wird. Es ist sogar denkbar, dass keine strömungstechnischen Elemente eingesetzt werden und die Änderung der strömungstechnischen Kenngröße nur durch die strömungstechnischen Eigenschaften des Messstreckenabschnitts und/oder der Messmischeinrichtung bedingt ist.

Bei einer möglichen Weiterbildung der Erfindung weist die Druckmesseinrichtung einen ersten Drucksensor auf, wobei der erste Drucksensor vor dem Messstreckenabschnitt und/oder in dem Zuführungsabschnitt angeordnet ist. Ferner weist die Druckmesseinrichtung einen zweiten Drucksensor auf, wobei der zweite Drucksensor nach dem Messstreckenabschnitt und/oder in dem Abführabschnitt angeordnet ist. Über die beiden Drucksensoren kann dies Druckänderung in dem Messstreckenabschnitt ermittelt werden. Bei einer alternativen Ausgestaltung wird der Druck nur an einer Stelle gemessen; ebenfalls ist eine Anordnung von mehr als zwei Druckmessstellen möglich.

Es kann vorgesehen sein, dass nur eine spezifizierte und/oder konditionierte Rücklaufzementsuspension in dem Messaufbau verwendet wird. Zur Überprüfung von Randbedingungen und/oder zur Verbesserung der Erfassung der rheologischen Eigenschaften können optional ergänzend mindestens eine, einige oder alle der nachfolgenden Hilfsmessgrößen zur Bestimmung der mindestens einen Qualitätskennzahl aufgenommen werden. Hierzu weist die Messvorrichtung mindestens eine, einige oder alle der nachfolgenden Sensoreinrichtungen auf:
Die Messvorrichtung weist optional einen Dichtesensor zur Messung der Dichte der Rücklaufzementsuspension und/oder Testsuspension als eine erste Hilfsmessgröße auf. Vorzugsweise ist der Dichtesensor als ein Coriolis-Messsystem ausgebildet, da dieses Messsystem gerade bei feststoffreichen und aggressiven Messstoffen Vorteile ergibt. Die Dichte der Rücklaufzementsuspension variiert infolge der Aufnahme von Bodenanteilen und Aufnahme von Porenwasser/Abgabe von Filtratwasser im Baugrund. Soweit nicht anders sichergestellt werden kann, dass die Rücklaufzementsuspension und/oder Testsuspension eine konstante und/oder eine bekannte Dichte aufweist, kann diese über den Dichtesensor erfasst werden und gegebenenfalls bei der Schätzung und/oder Bestimmung der mindestens einen Qualitätskennzahl berücksichtigt werden.

Die Messvorrichtung weist optional einen Temperatursensor zur Messung der Temperatur der Rücklaufzementsuspension und/oder der Testsuspension als eine zweite Hilfsmessgröße auf. Die Temperatur beeinflusst die rheologischen Eigenschaften der Rücklaufzementsuspension und/oder der Testsuspension. Soweit nicht anders sichergestellt werden kann, dass die Rücklaufzementsuspension und/oder Testsuspension eine konstante und/oder eine bekannte Temperatur aufweist, kann diese über den Temperatursensor erfasst werden und gegebenenfalls bei der Schätzung und/oder Bestimmung der mindestens einen Qualitätskennzahl berücksichtigt werden.

Die Messvorrichtung weist optional einen Leitfähigkeitssensor zur Messung der elektrischen Leitfähigkeit der Rücklaufzementsuspension und/oder der Testsuspension als eine dritte Hilfsmessgröße auf. Die Messvorrichtung weist optional einen pH-Sensor zur Messung des pH-Werts der Rücklaufzementsuspension und/oder der Testsuspension als eine vierte Hilfsmessgröße auf. Es ist aus anderen bautechnischen Anwendung bekannt, dass die beiden chemischen Parameter elektrische Leitfähigkeit und pH-Wert durch den Zementanteil beeinflusst werden.

Um sicherzustellen, dass die Rücklaufzementsuspension und das Zusatzmittel kontrolliert in die Messmischeinrichtung zugeführt werden, ist es bevorzugt, dass Durchflussratensensoren zur Messung der Volumenströme der Rücklaufzementsuspension und/oder des Zusatzmittels vorgesehen sind.

Es kann vorgesehen sein, dass die Auswerteeinrichtung ausgebildet ist, in Abhängigkeit von mindestens einer, einigen oder allen Hilfsmessgrößen die mindestens eine Qualitätskennzahl zu schätzen und/oder zu bestimmen.

Die Auswerteeinrichtung weist optional ein künstliches neuronales Netz auf. Insbesondere ist als künstliches neuronales Netz ein tiefes künstliches neuronales Netz zu verstehen. Das künstliche neuronale Netz weist Knoten auf, wobei die Knoten beispielsweise Gewichtungen zur Weiterverarbeitung eines Signals beschreiben. Das künstliche neuronale Netz ist eingelernt und/oder einlernbar, basierend auf der Hauptmessgröße und optional ergänzend mindestens einer, einige oder aller Hilfsmessgrößen die mindestens eine Qualitätskennzahl zu bestimmen. Beispielsweise ist das künstliche neuronale Netz eingelernt, dass die Hauptmessgröße und optional ergänzend mindestens einer, einige oder aller Hilfsmessgrößen als mindestens ein Eingangssignal dem künstlichen neuronalen Netz bereitgestellt ist, und das künstliche neuronale Netz, insbesondere in den Schichten und/oder mittels der Knoten, die Hauptmessgröße und optional ergänzend mindestens einer, einige oder aller Hilfsmessgrößen bewertet und/oder auswertet, um darauf basierend die mindestens ein Qualitätskennzahl zu bestimmen. Das künstliche neuronale Netz ist insbesondere als ein Klassifikator zu verstehen. Das künstliche neuronale Netz kann ein eingelerntes künstliches neuronales Netz darstellen, alternativ und/oder ergänzend kann das künstliche neuronale Netz im Verlauf des Betriebs der Auswerteeinrichtung weiterhin dazulernen. Das künstliche neuronale Netz ist hinsichtlich der Topologie vorzugsweise als ein mehrschichtiges Perzeptron ausgebildet.

Bei einer bevorzugten Ausgestaltung der Erfindung ist das Zusatzmittel als ein Beschleuniger und/oder als ein Stabilisierer, beispielsweise auch als ein Gemisch von Beschleuniger und Stabilisierer ausgebildet. Insbesondere ist das Zusatzmittel ausgebildet, ausschließlich oder zumindest maßgeblich mit dem Zementanteil zu interagieren. Vorzugsweise weist die zweite Zuführungseinrichtung das Zusatzmittel auf.

Als Stabilisierer, insbesondere chemische Stabilisierer, ist das Zusatzmittel als Polymer ausgebildet oder umfasst diese, wobei die Polymere die Viskosität der Rücklaufzementsuspension beeinflussen. Alternativ oder ergänzend können diese als Viskositätsmodifizier oder Viscosity Modifying Agents (VMA) bezeichnet werden. Nachdem die Stabilisierer eine Interaktion mit der flüssigen Phase, also dem Wasser, oder der festen Phase, also dem Zement- und/oder Bodenanteil, oder auch mit beiden Phasen umsetzen, ist es bevorzugt, dass der Stabilisierer ausgebildet ist, vorrangig mit dem Zement- und/oder Bodenanteil zu interagieren.

In der Ausbildung als Beschleuniger kann das Zusatzmittel als Erhärtungsbeschleuniger oder als Erstarrungsbeschleunigers ausgebildet sein. Bei Erhärtungsbeschleunigern wird die Anfangsfestigkeit von Betonen bzw. der Rücklaufzementsuspension erhöht. Mehr bevorzugt sind dagegen Erstarrungsbeschleuniger, welche die Zeit verringern bis die Rücklaufzementsuspension vom plastischen in den festen Zustand übergeht. Hierzu zählen beispielsweise auch die sogenannten Spritzbetonbeschleuniger. Diese Erstarrungsbeschleuniger erscheinen besonders geeignet für den Messaufbau.

Besonders bevorzugt ist das Zusatzmittel als Wasserglas, insbesondere als AlkaliWasserglas ausgebildet. Im Speziellen ist das Zusatzmittel als Natronwasserglas realisiert oder umfasst dieses
Das Natronwasserglas hat den Vorteil, dass dieses die Rücklaufzementsuspension innerhalb von weniger als 5 Minuten ansteift, es sich kein frühzeitiges Entmischen und keine Klumpenbildung ergibt und dass eine Stabilität bei weiterer Durchmischung vorherrscht.

Ein weiterer unabhängiger Gegenstand der Erfindung betrifft eine Baustellenanordnung mit den Merkmalen des Anspruchs 11. Die Baustellenanordnung dient insbesondere zur Umsetzung des Bohrverfahrens und/oder Düsenstrahlverfahrens zur Bodenstabilisierung wie dies zuvor beschrieben wurde.

Die Baustellenanordnung weist eine Aufnahmevorrichtung zur Aufnahme von Rücklaufzementsuspension aus einem Bohrloch auf. Vorzugsweise ist die Rücklaufzementsuspension wie zuvor beschrieben ausgebildet und erzeugt.

Die Baustellenanordnung weist eine Versorgungsmischeinrichtung auf, wobei diese einen ersten Zulauf für eine Zementsuspension, insbesondere eine frische Zementsuspension und einen zweiten Zulauf für die Rücklaufzementsuspension auf. Ferner weist die Versorgungsmischeinrichtung einen Ablauf zur Versorgung einer Bohrpumpeneinrichtung für ein Bohrgerät. Die Versorgungsmischeinrichtung ist ausgebildet, die Zementsuspension und die Rücklaufzementsuspension in einem Mischverhältnis, insbesondere in einem einstellbaren Mischverhältnis, zu einer Mischzementsuspension zu mischen. Die Mischzementsuspension wird über den Ablauf ausgegeben und insbesondere der Bohrpumpeneinrichtung für das Bohrgerät zugeführt.

Ferner weist die Baustellenanordnung einen Messaufbau für die Rücklaufzementsuspension auf, wobei der Messaufbau eine erste und eine zweite Zuführungseinrichtung aufweist, wie diese zuvor schon beschrieben wurden.

Ferner weist der Messaufbau eine Messmischeinrichtung zur Vermischung der Rücklaufzementsuspension mit dem Zusatzmittel zu einer Testsuspension, wobei die Messmischeinrichtung, dass Zusatzmittel und die Testsuspension so ausgebildet ist, wie dies zuvor schon beschrieben wurde.

Zudem weist der Messaufbau einen Messstreckenabschnitt zur Führung der Testsuspension auf, wobei diese ausgebildet ist, wie dieser zuvor beschrieben wurde. Der Messaufbau umfasst auch eine Messvorrichtung, wie diese zuvor beschrieben wurde.

Der Messaufbau weist eine Auswerteeinrichtung auf oder ist mit dieser datentechnisch verbunden, wobei die Auswerteeinrichtung insbesondere konstruktiv ausgebildet ist wie die zuvor beschriebene Auswerteeinrichtung. Die Auswerteeinrichtung ist ausgebildet, in Abhängigkeit der Hauptmessgröße mindestens eine Qualitätskennzahl für die Rücklaufzementsuspension zu schätzen und/oder zu bestimmen.

Ferner weist der Messaufbau eine Kontrolleinrichtung auf, wobei die Kontrolleinrichtung als ein Softwaremodul, als eine elektronische Datenverarbeitungseinrichtung, insbesondere als ein Computer, Mikrocontroller etc., ausgebildet sein kann. Die Kontrolleinrichtung ist ausgebildet das Mischverhältnis in der Versorgungsmischeinrichtung in Abhängigkeit der mindestens einen Qualitätskennzahl einzustellen. Wie bereits erläutert, kann die mindestens eine Qualitätskennzahl zum Beispiel als eine qualitative Beschreibung der Qualität der Rücklaufzementsuspension ausgebildet sein. Die Qualitätskennzahl ist ausgebildet, das Mischverhältnis in der Versorgungsmischeinrichtung über die Kontrolleinrichtung bestimmen zu können. Insbesondere bestimmt die Kontrolleinrichtung das Mischverhältnis derart, so dass die Mischzementsuspension einsatzfähig ist für das Düsenstrahlverfahren zur Bodenstabilisierung wie dies zuvor beschrieben wurde.

Vorzugsweise ist der Messaufbau nach einem der vorhergehenden Ansprüche bzw. wie zuvor beschrieben ausgebildet.

Bei einer bevorzugten Realisierung der Erfindung ist die Steuereinrichtung ausgebildet, die Qualitätskennzahl in Echtzeit bereitzustellen und/oder die Kontrolleinrichtung ausgebildet, die Versorgungsmischeinrichtung in Echtzeit zu kontrollieren. Die Echtzeitanforderung wird insbesondere dahingehend ausgelegt, dass alle Komponenten so schnell reagieren können, dass die Mischzementsuspension das eingestellte Mischverhältnis aufweist, so dass die Mischzementsuspension einsatzfähig ist.

Bei einer möglichen Weiterbildung der Erfindung weist die Aufnahmevorrichtung eine Separationseinrichtung zur Separierung zumindest eines Teils der Bodenanteile von der Rücklaufzementsuspension auf. Beispielsweise ist die Separationseinrichtung als ein Filter ausgebildet, welcher grobe Bodenanteile aussiebt. Besonders bevorzugt ist der Messaufbau strömungstechnisch hinter der Separationseinrichtung angeordnet.

Diese Ausgestaltung hat den Vorteil, dass der Messaufbau nicht durch grobe Bodenanteile blockiert ist. Ferner kann die Rücklaufzementsuspension besser für die Versorgungsmischeinrichtung verwendet werden.

Nachdem in dem Messaufbau nur ein Teil der Rücklaufzementsuspension quasi als eine Stichprobe überprüft werden muss, ist es prinzipiell möglich, dass der Messstreckenabschnitt in einer Lagereinrichtung mündet, so dass die Testsuspension zum Beispiel entsorgt wird. Besonders bevorzugt ist der Messstreckenabschnitt jedoch als ein Bypassabschnitt zwischen der Aufnahmevorrichtung gegebenenfalls mit der Separationseinrichtung und der Versorgungsmischeinrichtung angeordnet. Damit bildet die Aufnahmevorrichtung die erste Zuführungseinrichtung. Diese Weiterbildung hat den Vorteil, dass auch die zur Messung verwendete Testsuspension nicht entsorgt werden muss, sondern dem Verfahren wieder zugeführt wird.

Bei einer bevorzugten Weiterbildung der Erfindung weist die Baustellenanordnung die Bohrpumpeneinrichtung sowie das Bohrgerät auf. Das Bohrgerät kann beispielsweise als eine Drucklanze oder als ein Bohrer ausgebildet sein.

Ein weiterer Gegenstand der Erfindung wird durch ein Verfahren zur Herstellung einer Mischzementsuspension, insbesondere für ein Bodenverfahren und/oder für ein Düsenstrahlverfahren, im Speziellen für eine Bodenstabilisierung, mit den Merkmalen des Anspruchs 15 gebildet, welches besonders bevorzugt mit dem zuvor beschriebenen Messaufbau oder mit der zuvor beschriebenen Baustellenanordnung durchgeführt wird. Das Bohrverfahren und/oder Düsenstrahlverfahren wurde bereits zuvor beschrieben.

### Das Verfahren umfasst die folgenden Schritte:

Mischen einer Rücklaufzementsuspension mit einem Zusatzmittel zur Erzeugung einer Testsuspension, wobei das Zusatzmittel insbesondere wie zuvor beschrieben ausgebildet ist.

Messung von mindestens einer Hauptmessgröße, wobei die Hauptmessgröße abhängig von den rheologischen Eigenschaften der Testsuspension ist, wie dies insbesondere bereits zuvor beschrieben wurde. Bestimmung mindestens einer Qualitätskennzahl in Abhängigkeit der Hauptmessgröße, wie dies insbesondere bereits beschrieben wurde. Mischen der Rücklaufzementsuspension mit einer Zementsuspension in einem Mischverhältnis zu einer Mischzementsuspension, wobei das Mischverhältnis in Abhängigkeit der Qualitätskennzahl eingestellt wird, wie dies insbesondere bereits zuvor beschrieben wurde.

Weitere Merkmale, Vorteile und Wirkungen der Erfindung ergeben sich aus der nachfolgenden Beschreibung bevorzugter Ausführungsbeispiele der Erfindung sowie der beigefügten Figuren. Diese zeigen:
Figur 1 ein Blockdiagramm von einer Baustellenanordnung als ein Ausführungsbeispiel der Erfindung;
Figur 2 Blockdiagramm von einem Messaufbau der Baustellenanordnung als ein weiteres Ausführungsbeispiel der Erfindung.

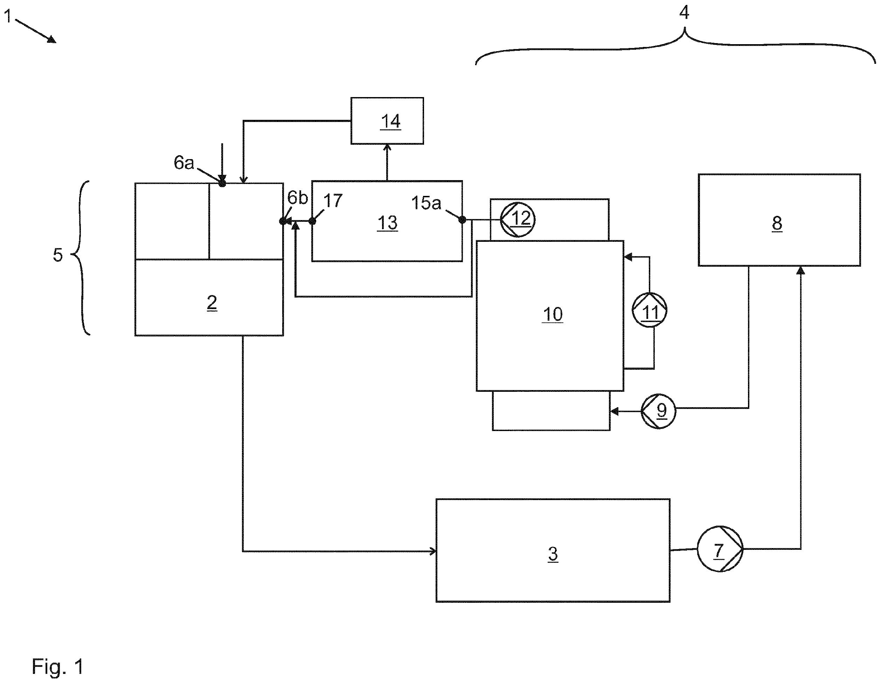

Die Figur 1 zeigt ein schematisches Blockdiagramm von einer Baustellenanordnung 1 als ein erstes Ausführungsbeispiel der Erfindung. Die Baustellenanordnung 1 hat zum Beispiel die Funktion eine Bodenstabilisierung durchzuführen, wobei über ein Düsenstrahlverfahren (DSV) anstehendes Bodenmaterial aus dem Erdboden mit dem Düsenstrahlverfahren aufgeschnitten wird. Alternativ hierzu kann die Baustellenanordnung 1 ein beliebiges Bohrverfahren umsetzen. Als Strahlmaterial und/oder Bohrmaterial wird eine zementhaltige Suspension verwendet. Bei dem Ausführungsbeispiel wird eine Mischzementsuspension als die zementhaltige Suspension verwendet. Die zementhaltige Suspension wird mit dem aufgeschnittenen Bodenmaterial vermischt, wobei das vermischte Material aushärtet und die Bodenstabilisierung bildet. Allerdings wird mit der zementhaltigen Suspension ein zusätzliches Volumen in den Boden eingebracht, welches als Rücklaufzementsuspension aus der Bohrung, insbesondere aus dem Bohrlochmund, austritt oder entnommen wird. Auch bei dem beliebigen Bohrverfahren entsteht die Rücklaufzementsuspension.

Die Mischzementsuspension wird über eine Bohrpumpeneinrichtung 2 zu einem Bohrgerät 3 geleitet, welches das Düsenstrahlverfahren oder das beliebige Bohrverfahren durchführt. Das Bohrgerät 3 kann bei dem Düsenstrahlverfahren beispielsweise als eine Düsenlanze ausgebildet sein, wobei die Mischzementsuspension mit Hochdruck, insbesondere mit einem Druck größer als 100 bar, ausgestoßen wird, um das anstehende Bodenmaterial aufzuschneiden.

Die Baustellenanordnung 1 weist eine Aufnahmevorrichtung 4 auf, welche den Rücklauf aus einem Bohrloch des Bohrgeräts 3 als Rücklaufzementsuspension aufnimmt. In der Rücklaufzementsuspension sind somit Zementanteile von der Mischzementsuspension sowie Bodenanteile aus dem Boden des Bohrlochs enthalten.

Die Rücklaufzementsuspension wird - gegebenenfalls nach einer Aufarbeitung - zu einer Versorgungsmischeinrichtung 5 geleitet. Die Versorgungsmischeinrichtung 5 weist einen ersten Zulauf 6 a auf, wobei der erste Zulauf 6 a mit einem Reservoir für eine insbesondere angemischte oder frische Zementsuspension strömungstechnisch verbunden ist, so dass die Zementsuspension in die Versorgungsmischeinrichtung 5 geführt werden kann. Ferner weist die Versorgungsmischeinrichtung einen zweiten Zulauf 6 b auf, wobei der zweite Zulauf 6 b strömungstechnisch mit der Aufnahmevorrichtung 4 verbunden ist, so dass die Rücklaufzementsuspension in die Versorgungsmischeinrichtung 5 geführt werden kann. In der Versorgungsmischeinrichtung 5 wird die Rücklaufzementsuspension mit der Zementsuspension in einem Mischverhältnis zu der Mischzementsuspension gemischt und nachfolgend gerührt, wobei die Mischzementsuspension dann der Bohrpumpeneinrichtung 2 zugeführt wird.

Die Zementsuspension wird somit mit der rezirkulierten Rücklaufzementsuspension vermischt und über die Bohrpumpeneinrichtung 2 dem Bohrgerät 3 zugeführt, wobei der Rücklauf aus dem Bohrloch in dem Kreislauf als Rücklaufzementsuspension wieder der Versorgungsmischeinrichtung 5 zugeführt wird.

Durch diesen Aufbau der Baustellenanordnung 1 entsteht durch den Rücklauf, also die Rücklaufzementsuspension, kein Material, welches entsorgt werden muss, vielmehr wird die Rücklaufzementsuspension in dem Kreislauf gehalten. Allerdings ist es möglich, dass verbrauchtes Material ausgesondert wird. Insgesamt betrachtet kann jedoch ein großer Teil des Rücklaufs und/oder der Rücklaufzementsuspension rezirkuliert und damit wiederverwendet werden.

Die Aufnahmevorrichtung 4 weist eine Rücklaufpumpeneinrichtung 7 auf, welche die Rücklaufzementsuspension aus dem Bohrbereich des Bohrgeräts 3 abgepumpt. Der Rücklauf, insbesondere die Rücklaufzementsuspension, wird optional in einen Pufferspeicher 8 zwischengelagert. Von dort wird die Rücklaufzementsuspension mit einer Beschickungspumpe 9 zu einer Separationseinrichtung 10 gepumpt, wobei die Separationseinrichtung 10 kann eine Beschickungspumpe 11 für einen Hydrozyklon aufweisen kann. In der Separationseinrichtung 10 werden grobe Bodenanteile aus der Rücklaufzementsuspension entfernt. Nachfolgend wird die Rücklaufzementsuspension über eine Beschickungspumpe 12 zu dem zweiten Zulauf 6b der Versorgungsmischeinrichtung 6 b gepumpt. Bei dem beliebigen Bohrverfahren kann auf die Separationseinrichtung 10 auch verzichtet werden.

Es ist bekannt, dass der Zementanteil in der Rücklaufzementsuspension abhängig von vielen Parametern und Faktoren ist. Somit ist es nicht möglich, ein statisches Mischverhältnis in der Versorgungsmischeinrichtung 5 einzustellen. Vielmehr muss das Mischverhältnis in Abhängigkeit der Qualität, insbesondere in Abhängigkeit des Zementanteils und/oder der Reaktivität, der Rücklaufzementsuspension eingestellt werden, so dass die Mischzementsuspension für die Baustelle einsatzfähig ist.

Die Baustellenanordnung 1 weist hierfür einen Messaufbau 13 sowie eine Kontrolleinrichtung 14 auf. Mit dem Messaufbau 13 wird mindestens eine Qualitätskennzahl für die Qualität der Rücklaufzementsuspension bestimmt oder abgeschätzt und an die Kontrolleinrichtung 14 übergeben. In Kenntnis der Qualität der über den ersten Zulauf 6a der Versorgungsmischeinrichtung 5 zugeführten Zementsuspension und in Kenntnis der Qualität der Rücklaufzementsuspension durch die Qualitätskennzahl kann die Kontrolleinrichtung 14 das Mischverhältnis zwischen der Zementsuspension und der Rücklaufzementsuspension einstellen, so dass die resultierende Mischzementsuspension einsatzfähig für die Baustelle ist. Ist somit der Zementanteil und/oder die Reaktivität der Rücklaufzementsuspension niedrig, so kann der Zementsuspension nur wenig Rücklaufzementsuspension beigemischt werden, um eine einsatzfähige Mischzementsuspension zu erhalten. Ist dagegen der Zementanteil und/oder die Reaktivität der Rücklaufzementsuspension hoch, so kann der Zementsuspension mehr Rücklaufzementsuspension beigemischt werden, um eine einsatzfähige Mischzementsuspension zu erhalten.

Die Baustellenanordnung 1 ist mit dem Messaufbau 13 echtzeitfähig, so dass die über die Aufnahmevorrichtung 4 bereitgestellte Rücklaufzementsuspension in Echtzeit beurteilt und das Mischverhältnis ebenfalls in Echtzeit eingestellt werden kann. Damit entfällt die Notwendigkeit, die Rücklaufzementsuspension zunächst zwischen zu lagern und nicht nur zwischen zu puffern, so dass aufwändige Lagerbehälter oder sogar ein Abtransport der Rücklaufzementsuspension von der Baustelle, wie dies bislang der Fall war, nicht notwendig ist.

Die mindestens eine Qualitätszahl kann als eine physikalische Größe ausgebildet sein, es kann sich jedoch auch um eine qualitative Größe oder um qualitative Größen handeln. Der Messaufbau 13 ist strömungstechnisch hinter der Aufnahmevorrichtung 4 angeordnet. Durch den Messaufbau 13 wird mindestens ein Teil der Rücklaufzementsuspension geleitet, wobei der Messaufbau 13 bei dem Ausführungsbeispiel in der Figur 1 als ein Bypass zu der Leitung zwischen der Aufnahmevorrichtung 4 und der Versorgungsmischeinrichtung 5 ausgebildet ist. Dadurch, dass der Messaufbau 13 in einem Bypass zu der Versorgungsleitung angeordnet ist, geht nahezu kein Material von der Rücklaufzementsuspension verloren. Alternativ wäre es auch möglich, dass der Anteil von der Rücklaufzementsuspension, der den Messaufbau 5 durchfließt, nachfolgend abgesondert wird.

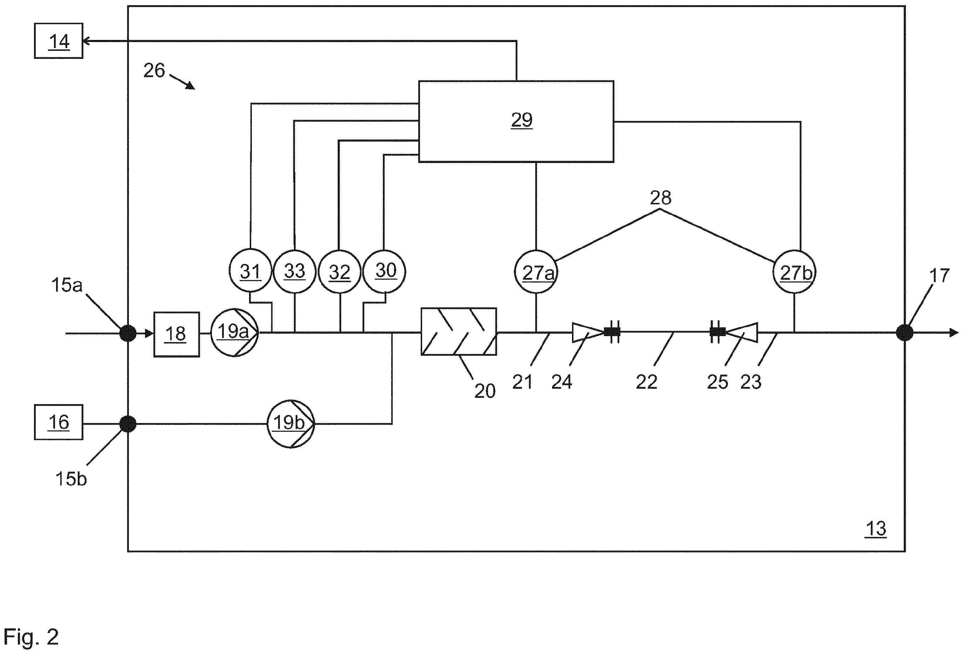

In der Figur 2 ist eine Blockdarstellung des Messaufbaus 13 dargestellt. Der Messaufbau 13 hat die Funktion, rheologische Parameter der Rücklaufzementsuspension bzw. eine Änderung von rheologischen Parametern der Rücklaufzementsuspension aufgrund einer chemischen Modifikation der Rücklaufzementsuspension zu der Testsuspension zu erfassen und als Qualitätsmaßstab für die Rücklaufzementsuspension, insbesondere den Zementanteil und die Reaktivität der Rücklaufzementsuspension, zu bestimmen. Aus dem Qualitätsmaßstab wird nachfolgend die mindestens eine Qualitätskennzahl abgeleitet.

Der Messaufbau 13 weist eine erste Zuführungseinrichtung 15 a zur Zuführung der Rücklaufzementsuspension auf, wobei die erste Zuführungseinrichtung 15 a beispielsweise als Einleitung ausgebildet ist und mit am Ausgang der Aufnahmevorrichtung 4 strömungstechnisch verbunden ist. Ferner weist der Messaufbau 13 eine zweite Zuführungseinrichtung 15 b auf, wobei die zweite Zuführungseinrichtung 15 b mit einem Reservoir 16 verbunden ist oder dieses umfasst, wobei in dem Reservoir 16 ein Zusatzmittel angeordnet ist. Ein Ablauf 17 kann - wie in der Figur 1 dargestellt - mit der Versorgungsmischeinrichtung 5 strömungstechnisch verbunden sein, so dass der Messaufbau 13 als eine Bypassanordnung ausgebildet ist, alternativ führt der Ablauf 17 zu einem nicht dargestellten Entsorgungsbehälter. Optional ist in dem Messaufbau 13 ein Rücklaufbehälter 18 mit der Rücklaufzementsuspension angeordnet, welcher einen Teil der ersten Zuführungseinrichtung 15 a bildet und als ein Pufferspeicher ausgebildet ist. Nachdem jedoch der Messaufbau 13 in Bezug auf die parallel zu der Versorgungsmischeinrichtung 5 geführte Rücklaufzementsuspension in Echtzeit arbeiten soll, ist es bevorzugt, dass der Rücklaufbehälter 18 sehr klein ausgebildet ist oder vollständig entfällt.

Hinter der ersten Zuführungseinrichtung 15 a ist eine erste Dosierpumpeneinrichtung 19 a angeordnet, welche die Rücklaufzementsuspension pumpt. Hinter der zweiten Zuführungseinrichtung 15 b ist eine zweite Dosierpumpeneinrichtung 19 b angeordnet, welche das Zusatzmittel pumpt. Die Pumpen sind jeweils als Exzenterschneckenpumpen ausgebildet, die sehr pulsationsarm arbeiten.

Nachfolgend ist eine Messmischeinrichtung 20 angeordnet, wobei die Messmischeinrichtung 20 das Gemisch aus Rücklaufzementsuspension und Zusatzmittel zu einer Testsuspension insbesondere homogen mischt. Die Messmischeinrichtung 20 ist insbesondere als ein Statikmischer ausgebildet.

Über einen Zuführungsabschnitt 21 wird die Testsuspension von der Messmischeinrichtung 20 zu einem Messstreckenabschnitt 22 und nachfolgend zu einem Abführungsabschnitt 23 geleitet, wobei der Abführungsabschnitt 23 strömungstechnisch mit dem Ablauf 17 verbunden ist. Der Zuführungsabschnitt 21, der Messstreckenabschnitt 22 und der Abführungsabschnitt 23 sind strömungstechnisch miteinander ohne Abflüsse und Zuflüsse verbunden.

Der Messstreckenabschnitt 22 bildet eine Messstrecke mit verengtem Strömungsquerschnitt. Hierzu ist vor dem Messstreckenabschnitt 22 und hinter dem Zuführungsabschnitt 21 und/oder zwischen Zuführungsabschnitt 21 um Messstreckenabschnitt 22 eine Eingangsdrossel 24 angeordnet, welche den Leitungsquerschnitt verringert. Ferner ist hierzu nach dem Messstreckenabschnitt 22 und vor dem Abführungsabschnitt 23 eine Ausgangsdrossel 25 angeordnet, welche den Leitungsquerschnitt vergrößert. Somit ist der Leitungsquerschnitt in dem Messstreckenabschnitt 22 kleiner als der Leitungsquerschnitt in dem Zuführungsabschnitt 21 und kleiner als der Leitungsquerschnitt in dem Abführungsabschnitt 23.

Funktional betrachtet werden die strömungsbedingten Druckänderungen, insbesondere Druckerhöhungen in einem Strom der Rücklaufzementsuspension, nach Zugabe des Zusatzmittels mittels einer Differenzdruckmessung gemessen. Prinzipiell gilt, dass die Druckänderungen in Folge von Ansteifen, mit einhergehender Erhöhung der maßgeblichen rheologischen Kenngrößen Fließfähigkeit/Viskosität/Fließgrenze umso ausgeprägter sind, desto größer die Reaktivität und/oder der Zementanteil sind. Somit wird innerhalb einer definierten Messapparatur die Druck- und Suspensionsveränderungen durch mengengesteuerte Zugabe eines Zusatzmittels gemessen und dokumentiert/registriert.

Das Zusatzmittel ist insbesondere als ein Erstarrungsbeschleuniger, wie zum Beispiel Natronwasserglas, ausgebildet. Durch die Mischung von dem Zusatzmittel mit der Rücklaufzementsuspension zur Testsuspension wird erreicht, dass sich die rheologischen Eigenschaften der Rücklaufzementsuspension ändern, wobei aufgrund der Änderung der rheologischen Eigenschaften auf die Qualität, insbesondere den Zementanteil und/oder die Reaktivität, im Speziellen den reaktiven Zementanteil der Rücklaufzementsuspension geschlossen werden kann. Der Messaufbau 13 weist eine Messvorrichtung 26 mit einer Druckmesseinrichtung 28 auf, wobei die Druckmesseinrichtung 28 den Differenzdruck als eine Hauptmessgröße zwischen einem ersten Messpunkt in dem Zuführungsabschnitt 21 vor dem Messstreckenabschnitt 22 und einem zweiten Messpunkt in dem Abführungsabschnitt 23 nach dem Messstreckenabschnitt 22 aufnimmt. Die Druckmesseinrichtung 28 weist einen ersten Drucksensor 27 a auf, wobei der erste Drucksensor 27 a strömungstechnisch vordem Messstreckenabschnitt 22 und insbesondere unmittelbar vor dem Messstreckenabschnitt 22, jedoch vor der Eingangsdrossel 24 angeordnet ist. Die Druckmesseinrichtung 28 weist einen zweiten Drucksensor 27 b auf, wobei der zweite Drucksensor 27 b strömungstechnisch nach dem Messstreckenabschnitt 22 und insbesondere unmittelbar nach dem Messstreckenabschnitt 22, jedoch nach der Ausgangsdrossel 25 angeordnet ist.

Der Messaufbau 13 weist eine Auswerteeinrichtung 29 auf, wobei die Auswerteeinrichtung 29 ausgebildet ist, auf Basis des Druckunterschieds als Hauptmessgröße zwischen dem ersten und dem zweiten Messpunkt und/oder auf Basis des Druckverlustes in dem Messstreckenabschnitt 22 eine Qualitätskennzahl für die Testsuspension und damit für die Rücklaufzementsuspension zu schätzen und/oder zu bestimmen. Die Qualitätskennzahl ist ein Qualitätsmaß für die Reaktivität und/oder den Zementanteil in der Testsuspension bzw. in der Rücklaufzementsuspension.

Die Auswerteeinrichtung 29 ist mit der Kontrolleinrichtung 14 datentechnisch verbunden oder programmtechnisch und/oder schaltungstechnisch als eine gemeinsame Einrichtung ausgebildet und liefert die Qualitätskennzahl in einer beliebigen Form an die Kontrolleinrichtung 14. Die Kontrolleinrichtung 14 stellt das Mischverhältnis in der Versorgungsmischeinrichtung 5 in Abhängigkeit der Qualitätskennzahl ein. Der Zusammenhang zwischen Druckänderung oder Druckgröße, Reaktivität, Zementanteil und Mischverhältnis kann experimentell über Kennlinienfelder aufgenommen und eingestellt werden. Beispielsweise können Look-Up-Tables oder andere Regelwerke in der Kontrolleinrichtung 14 abgelegt sein.

Alternativ hierzu kann ein künstliches neuronales Netz in der Kontrolleinrichtung 14 genutzt werden, wobei der Druckunterschied eine Eingangsgröße in das künstliche neuronale Netz bildet.

Die Qualität der Rücklaufzementsuspension ist neben den rheologischen Eigenschaften von anderen Parametern abhängig. Somit können verschiedene Sensoren verwendet werden, um Hilfsmessgrößen aufzunehmen. Die Sensoren sind anwendungsabhängig einzusetzen und nur dann notwendig, wenn sich der jeweilige Parameter ändert. Beispielsweise kann es sinnvoll sein, einen Dichtesensor 30, einen Temperatursensor 31, einen Leitfähigkeitssensor 32 und/oder einen pH-Sensor 33 einzusetzen. Diese Sensoren können entweder hinter der Dosierpumpeneinrichtung 19 a in die Leitung integriert werden oder es erfolgt eine ggf. händische Messung im Rücklaufbehälter 18. Ferner ist es möglich, die Durchflussrate mit entsprechenden Durchflussratensensoren zu kontrollieren.

Die aus den genannten Sensoren 30, 31, 32, 33 erfassten Hilfsmessgrößen können ebenfalls der Auswerteeinrichtung 29, insbesondere dem künstlichen neuronalen Netz zugeführt werden, um die Qualitätskennzahl besser bestimmen zu können.

### Bezugszeichenliste

- 1: Baustellenanordnung
- 2: Bohrpumpeneinrichtung für das Bohrgerät 3
- 3: Bohrgerät
- 4: Aufnahmevorrichtung
- 5: Versorgungsmischeinrichtung
- 6a: erster Zulauf der Versorgungsmischeinrichtung
- 6b: zweiter Zulauf der Versorgungsmischeinrichtung
- 7: Rücklaufpumpeneinrichtung
- 8: Pufferspeicher
- 9: Beschickungspumpe für
- 10: Separationseinrichtung
- 11: Beschickungspumpe für Hydrozyklon
- 12: Beschickungspumpe für Versorgungsmischeinrichtung
- 13: Messaufbau
- 14: Kontrolleinrichtung
- 15a: erste Zuführungseinrichtung des Messaufbaus
- 15b: zweite Zuführungseinrichtung des Messaufbaus
- 16: Reservoir
- 17: Ablauf
- 18: Rücklaufbehälter
- 19a: erste Dosierpumpeneinrichtung
- 19b: zweite Dosierpumpeneinrichtung
- 20: Messmischeinrichtung
- 21: Zuführungsabschnitt
- 22: Messstreckenabschnitt
- 23: Abführungsabschnitt
- 24: Eingangsdrossel
- 25: Ausgangsdrossel
- 26: Messvorrichtung
- 27a: erster Drucksensor
- 27b: zweiter Drucksensor
- 28: Druckmesseinrichtung
- 29: Auswerteeinrichtung
- 30: Dichtesensor
- 31: Temperatursensor
- 32: Leitfähigkeitssensor
- 33: pH-Sensor

## Patentansprüche

1. Messaufbau (13) für eine Rücklaufzementsuspension, wobei die Rücklaufzementsuspension Zementanteile und Bodenanteile aufweist,
mit einer Messmischeinrichtung (20) zur Vermischung der Rücklaufzementsuspension mit einem Zusatzmittel zu einer Testsuspension,
mit einem Messstreckenabschnitt (22) zur Führung der Testsuspension,
mit einer Messvorrichtung (26) zur Messung von mindestens einer Hauptmessgröße an dem Messstreckenabschnitt (22),
mit einer Auswerteeinrichtung (29), wobei die Auswerteeinrichtung (29) ausgebildet ist, in Abhängigkeit der Hauptmessgröße mindestens eine Qualitätskennzahl für die Rücklaufzementsuspension zu schätzen und/oder zu bestimmen.

2. Messaufbau (13) nach Anspruch 1, **gekennzeichnet durch** eine erste Zuführungseinrichtung (15a) zur Zuführung der Rücklaufzementsuspension und/oder durch eine zweite Zuführungseinrichtung (15b) zur Zuführung des Zusatzmittels

3. Messaufbau (13) nach Anspruch 1 oder 2, **dadurch gekennzeichnet, dass** das Zusatzmittel zur Änderung der rheologischen Eigenschaften der Rücklaufzementsuspension durch Reaktion mit den Zementanteilen der Rücklaufzementsuspension ausgebildet ist und/oder dass die Hauptmessgröße abhängig von den rheologischen Eigenschaften der Testsuspension ist und/oder dass die mindestens eine Qualitätskennzahl die Reaktivität und/oder den Zementanteil der Rücklaufzementsuspension und/oder die Mischeignung der Rücklaufzementsuspension mit einer Zementsuspension zur Erzeugung einer einsatzfähigen Mischzementsuspension beschreibt.

4. Messaufbau (13) nach einem der vorhergehenden Ansprüche, **dadurch gekennzeichnet, dass** die Hauptmessgröße als eine strömungstechnische Kenngröße, insbesondere als eine Druckänderung und/oder Änderung der Strömungsgeschwindigkeit in dem Messstreckenabschnitt (22) ausgebildet ist.

5. Messaufbau (13) nach einem der vorhergehenden Ansprüche, **gekennzeichnet durch** einen Zuführungsabschnitt (21) zur Zuführung der Testsuspension zu dem Messstreckenabschnitt (22), und einen Abführungsabschnitt (23) zur Abführung der Testsuspension aus dem Messstreckenabschnitt (22),
wobei der Strömungsquerschnitt des Messstreckenabschnitts (22) an mindestens einer Stelle kleiner als der Strömungsquerschnitt des Zuführungsabschnitts (21) ausgebildet ist, und wobei der Strömungsquerschnitt des Messstreckenabschnitts (22) an mindestens einer Stelle kleiner als der Strömungsquerschnitt des Abführungsabschnitts (23) ausgebildet ist,
und/oder wobei die Strömungsgeschwindigkeit in dem Messstreckenabschnitt (22) größer als die Strömungsgeschwindigkeit in dem Zuführungsabschnitt (21) und wobei die Strömungsgeschwindigkeit in dem Messstreckenabschnitt (22) größer als die Strömungsgeschwindigkeit in dem Abführungsabschnitt (23) ist,
und/oder wobei zwischen dem Zuführungsabschnitt (21) und dem Messstreckenabschnitt (22) eine Eingangsdrossel (24) zur Verringerung des Strömungsquerschnitts und zwischen dem Messstreckenabschnitt (22) und dem Abführungsabschnitt (23) eine Ausgangsdrossel (25) zur Vergrößerung des Strömungsquerschnitts angeordnet ist,
wobei die Messvorrichtung (26) eine Druckmesseinrichtung (28) zur Bestimmung eines Druckunterschieds der Testsuspension vor und nach dem Messstreckenabschnitt (22) und/oder zwischen dem Zuführungsabschnitt (21) und dem Abführabschnitt (23) und/oder eine Strömungsgeschwindigkeitsmesseinrichtung zur Bestimmung der Strömungsgeschwindigkeit in dem Messstreckenabschnitt als die Hauptmessgröße aufweist.

6. Messaufbau (13) nach Anspruch 5, **dadurch gekennzeichnet, dass** die Druckmesseinrichtung (28) einen ersten Drucksensor (27a) aufweist, wobei der erste Drucksensor (27a) vor dem Messstreckenabschnitt (22) und/oder in dem Zuführungsabschnitt (21) angeordnet ist, und einen zweiten Drucksensor (27b) aufweist, wobei der zweite Drucksensor (27b) nach dem Messstreckenabschnitt (22) und/oder in dem Abführungsabschnitt (23) angeordnet ist.

7. Messaufbau (13) nach einem der vorhergehenden Ansprüche, **dadurch gekennzeichnet, dass** die Messvorrichtung (26) einen Dichtesensor (30) zur Messung der Dichte der Rücklaufzementsuspension und/oder der Testsuspension als eine erste Hilfsmessgröße aufweist und/oder dass die Messvorrichtung (26) einen Temperatursensor (31) zur Messung der Temperatur der Rücklaufzementsuspension und/oder der Testsuspension als eine zweite Hilfsmessgröße aufweist und/oder dass die Messvorrichtung (26) einen Leitfähigkeitssensor (32) zur Messung der elektrischen Leitfähigkeit Rücklaufzementsuspension und/oder der Testsuspension als eine dritte Hilfsmessgröße aufweist und/oder dass die Messvorrichtung (26) einen pH-Sensor (33) zur Messung eines pH-Werts der Rücklaufzementsuspension und/oder der Testsuspension als eine vierte Hilfsmessgröße aufweist, wobei die Auswerteeinrichtung (29) ausgebildet ist, in Abhängigkeit von mindestens einer, einigen oder allen Hilfsmessgrößen die mindestens eine Qualitätskennzahl zu schätzen und/oder zu bestimmen.

8. Messaufbau (13) nach einem der vorhergehenden Ansprüche, **dadurch gekennzeichnet, dass** die Auswerteeinrichtung (29) ein künstliches neuronales Netz aufweist, wobei das künstliche neuronale Netz als Eingangsparameter mindestens die Hauptmessgröße und optional ergänzend mindestens einen, einige oder alle Hilfsmessgrößen erhält und als Ausgangsparameter die mindestens eine Qualitätskennzahl der Rücklaufzementsuspension ausgibt.

9. Messaufbau (13) nach einem der vorhergehenden Ansprüche, **dadurch gekennzeichnet, dass** die zweite Zuführungseinrichtung (15b) das Zusatzmittel aufweist, wobei das Zusatzmittel als ein Beschleuniger und/oder als ein Stabilisierer ausgebildet ist.

10. Messaufbau (13) nach einem der vorhergehenden Ansprüche, **dadurch gekennzeichnet, dass** das Zusatzmittel als Wasserglas ausgebildet ist oder aufweist.

11. Baustellenanordnung (1)
mit einer Aufnahmevorrichtung (4) zur Aufnahme von Rücklaufzementsuspension aus einem Bohrloch, wobei die Rücklaufzementsuspension Zementanteile und Bodenanteile aufweist,
mit einer Versorgungsmischeinrichtung (5), wobei die Versorgungsmischeinrichtung (5) einen ersten Zulauf (6a) für eine Zementsuspension und einen zweiten Zulauf (6b) für die Rücklaufzementsuspension sowie einen Ablauf zur Versorgung einer Bohrpumpeneinrichtung (2) für ein Bohrgerät (3) aufweist, wobei die Versorgungsmischeinrichtung (5) die Zementsuspension und die Rücklaufzementsuspension in einem Mischverhältnis zu einer Mischzementsuspension mischt,
mit einem Messaufbau (13) vorzugsweise ausgebildet nach einem der vorhergehenden Ansprüche für die Rücklaufzementsuspension,
wobei der Messaufbau (13)
eine Messmischeinrichtung (20) zur Vermischung der Rücklaufzementsuspension mit einem Zusatzmittel zu einer Testsuspension,
einen Messstreckenabschnitt (22) zur Führung der Testsuspension,
eine Messvorrichtung (26) zur Messung von mindestens einer Hauptmessgröße in dem Messstreckenabschnitt (22) aufweist,
und eine Auswerteeinrichtung (29) umfasst oder mit dieser datentechnisch verbunden ist, wobei die Auswerteeinrichtung (29) ausgebildet ist, in Abhängigkeit der Hauptmessgröße mindestens eine Qualitätskennzahl für die Rücklaufzementsuspension zu schätzen und/oder zu bestimmen,
mit einer Kontrolleinrichtung (14), wobei die Kontrolleinrichtung (14) ausgebildet ist, das Mischverhältnis in der Versorgungsmischeinrichtung (5) in Abhängigkeit der mindestens eine Qualitätskennzahl einzustellen.

12. Baustellenanordnung (1) nach Anspruch 11, **dadurch gekennzeichnet, dass** Auswerteeinrichtung (29) ausgebildet ist, die Qualitätskennzahl in Echtzeit bereitzustellen und/oder die Kontrolleinrichtung (14) ausgebildet ist, die Versorgungsmischeinrichtung (5) in Echtzeit zu kontrollieren.

13. Baustellenanordnung (1) nach einem der Ansprüche 11 bis 12, **dadurch gekennzeichnet, dass** die Aufnahmevorrichtung (4) eine Separationseinrichtung (10) zur Separierung zumindest eines Teils der Bodenanteile von der Rücklaufzementsuspension aufweist.

14. Baustellenanordnung (1) nach einem der Ansprüche 11 bis 13, **dadurch gekennzeichnet, dass** der Messstreckenabschnitt (22) als ein Bypassabschnitt zwischen der Aufnahmevorrichtung (4) und der Versorgungsmischeinrichtung (5) ausgebildet ist und/oder dass die Aufnahmevorrichtung (4) die erste Zuführungseinrichtung (15a) bildet oder umfasst.

15. Baustellenanordnung (1) nach einem der Ansprüche 11 bis 14, **dadurch gekennzeichnet, dass** diese die Bohrpumpeneinrichtung (2) und/oder das Bohrgerät (3) umfasst.

16. Verfahren zur Herstellung einer Mischzementsuspension, insbesondere für ein Düsenstrahlverfahren oder ein Bohrverfahren, umfassend die Schritte:
Mischen einer Rücklaufzementsuspension, wobei die Rücklaufzementsuspension Zementanteile und Bodenanteile aufweist, mit einem Zusatzmittel zur Erzeugung einer Testsuspension;
Messung von mindestens einer Hauptmessgröße der Testsuspension;
Bestimmung mindestens einer Qualitätskennzahl in Abhängigkeit der Hauptmessgröße;
Mischen der Rücklaufzementsuspension mit einer Zementsuspension in einem Mischverhältnis zu der Mischzementsuspension, wobei das Mischverhältnis in Abhängigkeit der Qualitätskennzahl eingestellt ist.

17. Verwendung eines Zusatzmittels in dem Messaufbau (13) nach einem der Ansprüche 1 bis 10 und/oder in der Baustellenanordnung (1) nach einem der Ansprüche 11 bis 15 und/oder in dem Verfahren nach Anspruch 16.
